# FASCICULE DE BREVET EUROPEEN

(11) **EP 0 758 245 B1**
(45) Date de publication et mention de la délivrance du brevet: **19.03.2003**
(21) Numéro de dépôt: 95918047.2
(22) Date de dépôt: 24.04.1995
(51) Int. Cl.: A61K 31/7048, A61P 1/04

(54) **UTILISATION DE LA SPIRAMYCINE DANS DES DESORDRES GASTROINTESTINAUX DUS A H. PYROLI**
VERWENDUNG VON SPIRAMYEINE IN VON H.PYLORI VERURSACHTEN MAGEN UND DARM BESCHWERDEN
USE OF SPIRAMYCIN FOR TREATING GASTROINTESTINAL DISORDERS CAUSED BY H. PYLORI

(30) Priorité: 25.04.1994 FR 9404952
(43) Date de publication de la demande: 19.02.1997
(73) Titulaire: Aventis Pharma S.A., 92160 Antony (FR)
(72) Inventeur: FORFANG, Erik, T., N-1342 Jar (NO); LARSSEN, Sven-Erik, N-0751 Oslo (NO)
(86) Numéro de dépôt international: FR9500533
(87) Numéro de publication internationale: WO95028943

(56) Documents cités:
- J. GASTROENTEROL. HEPATOL., vol. 6, no. 2, 1991 pages 131-137, A.T.R. AXON 'Helicobacter pylori therapy: effect on peptic ulcer disease.'
- MED. DOSW. MIKROBIOL., vol. 45, no. 3, 1993 pages 339-343, J. BORYSIEWICZ ET AL. 'Drug resistance of strains of Helicobacter pylori isolated from patients with inflammation of stomach mucous membrane and peptic ulcer of stomach and duodenum.'
- D.J. Hardy et al., J. of Antimicrobial Chemotherapy (1988) 22, 631-636

## Description

La présente invention concerne une nouvelle application thérapeutique de la spiramycine éventuellement en association avec le métronidazole.

La spiramycine éventuellement en association avec le métronidazole peut être utile pour la préparation d'un médicament destiné à la prévention ou au traitement des désordres gastrointestinaux dans lesquels intervient *Helicobacter pylori.*

De nombreux cas d'ulcères peptiques sont identifiés chaque année. Ces ulcères sont habituellement traités par des agents anti-ulcères tels que des anti-H₂, des dérivés du bismuth ou des inhibiteurs de pompe à protons. Cependant il s'agit de traitements à long terme qui sont très coûteux, difficiles à faire accepter par les patients et dans lesquels de nombreuses rechutes sont observées.

*Helicobacter pylori,* isolé pour la première fois de la muqueuse gastrique en 1982, est responsable de gastropathies telles que des gastrites et des ulcères peptiques, et probablement aussi à l'origine de l'apparition de cancers gastriques. L'éradication d'*Helicobacter pylori* modifie largement l'importance des maladies ulcéreuses. Non seulement les ulcères disparaissent rapidement (environ 90 %), mais les cas de rechutes sont presque complètement éliminés.

Des traitements en vue d'éradiquer Helicobacter pilori au moyen de compositions comprenant un antibactérien et un agent anti-ulcère sont déjà mentionnés dans les demandes de brevet EP 206 625, EP 282 131, WO 92/04 898 et WO 93/21 920.

Cependant de nombreuses publications rapportent des manifestations d'intolérance qui apparaissent chez 20 à plus de 40 % des patients, la plupart du temps constitués de nausées et de diarrhées, mais aussi de douleurs abdominales. Ces effets secondaires proviennent de la nature des produits actuellement utilisés en clinique, des doses élevées qui doivent être administrées et de la durée des traitements.

De plus la majorité des thérapies efficaces consistent dans l'administration de 3 médicaments, ce qui est mal accepté par les patients. Ainsi le praticien est confronté au choix difficile entre la puissance du traitement, la limitation des effets secondaires et la médiocre acceptation du patient pour une telle médication.

La spiramycine est un agent antibactérien bien connu, isolé à partir de *Streptomyces ambofaciens* : US 2 943 023, US 2 978 380 et US 3 011 947.
Le métronidazole est un dérivé de nitro imidazole également connu (US 2 944 061) et doué d'une action parasiticide et antibactérienne.
L'association spiramycine/métronidazole commercialisée sous le nom Rodogyl® est connue pour son action sur les germes anaérobies de la flore bucco-dentaire.

Cependant l'activité de la spiramycine ou de ses associations avec le métronidazole ne laissait pas supposer qu'une action puisse être observée en clinique sur un germe difficile à éradiquer comme *Helicobacter pylori* pour lequel il est habituellement nécessaire d'employer des antibactériens beaucoup plus puissants, avec les conséquences que l'on connaît.

Il a maintenant été montré qu'il est possible d'obtenir l'éradication d'*Helicobacter pylori* par administration de spiramycine ou d'une association spiramycine/métronidazole, avec un taux d'effets secondaires tout à fait diminués par rapport aux autres traitements.

L'administration s'effectue avant, après ou simultanément à l'administration d'un agent anti-ulcère

Les agents anti-ulcères sont choisis indifféremment parmi les antiacides, les anti H₂ et les inhibiteurs de pompe à protons.

Les antiacides peuvent être notamment des dérivés du bismuth ou des combinaisons d'hydroxyde d'aluminium et d'hydroxyde de magnésium comme le Maalox®.

Les anti-H₂ peuvent être par exemple la ranitidine, la cimétidine, la famotidine .....

Les inhibiteurs de pompe à protons peuvent être par exemple l'oméprazole, le lansoprazole, le pantoprazole ....

Parmi les associations spiramycine/métronidazole, plus particulièrement préférée est l'association qui compose le Rodogyl® (association 750 000 U.I. spiramycine base / 125 mg métronidazole). Cette association préexistante permet de surcroît de n'administrer qu'une seule spécialité antibactérienne au cours du traitement.

L'activité a été mise en évidence dans un traitement de 10 jours chez des malades atteints d'ulcères peptiques.

25 patients souffrant d'ulcère peptiques ont été inclus selon les critères suivants :
inclusion : hommes et femmes de 18 à 80 ans motivés, souffrant d'ulcère bénin duodénal ou gastrique vérifié par endoscopie haute et ayant subi un test positif d'uréase à partir de tissu prélevé par biopsie, préalablement au traitement [G. Nysaeter, K. Berstad et coll., Tidsskr Nor Laegeforen, 112, 2397-9 (1992)];
exclusion : patients risquant de présenter des risques d'allergie à l'un des médicaments administrés, femmes enceintes ou sans contraception, patients présentant d'autres maladies gastrointestinales risquant de modifier l'interprétation du test, ainsi que patients recevant un autre anti-ulcère au moment de l'inclusion dans l'étude ou ayant rechuté après un traitement préalable en vue d'éradiquer *Helicobacter pylori.*

### TRAITEMENT :

La spiramycine (Rovamycine®) 1 500 000 U.I. et 150 mg de sous-nitrate de bismuth sous forme d'une solution à 1,5 % (10 ml) sont administrés tous les 2, 4 fois par jour (soit au total 6 millions d'U.I. de spiramycine et 600 mg de sous-nitrate de bismuth). 400 mg de métronidazole (Flagyl®) sont administrés 3 fois par jour. Le même traitement est poursuivi pendant 10 jours, la spiramycine et le métronidazole sont administrés pendant les repas et le bismuth entre les repas. Pendant les semaines qui suivent les patients ne reçoivent aucune autre médication anti-ulcère.

Un questionnaire concernant les effets secondaires a été remis à chaque patient. Le questionnaire est identique à celui remis lors de précédentes études d'éradication d'Hélicobacter pylori [K. Berstad et coll., Is there a place for antacids in treatment of *Helicobacter pylori* infection?, Scand. J. Gastroenterol., 27, 1006-1010 (1992)]. Les effets secondaires ont été classés comme "légers" (ne limitent pas les activités journalières habituelles), "modérés" (limitent les activités journalières dans une certaine mesure) et "sévères" (rendent les activités journalières impossibles).

### Eradication d'Helicobacter pylori et guérison des ulcères :

Un test de respiration et une nouvelle endoscopie sont effectués 4 semaines après l'arrêt du traitement. Dans le cas où un ulcère serait détecté, le traitement serait considéré comme un échec et le malade recevrait un traitement conventionnel. Des tests d'uréase à partir de tissu prélevé par biopsie, et de respiration à la ¹⁴C-urée [K. Bergstad et coll., Biometric evaluation of gastric urease activity in man, Scand. J. Gastroenterol., 27, 977-83 (1992)] sont réalisés.

### RESULTATS :

2 malades ayant refusé l'un de poursuivre le traitement jusqu'à la fin, l'autre de se soumettre aux tests, sont exclus de l'étude. Les résultats ci-après concernent les 23 autres malades.

7 femmes et 16 hommes d'age moyen 53,9 ans (allant de 31 à 81 ans) ont été inclus, chez lesquels la maladie durait en moyenne depuis 18,7 années, à savoir depuis 1 à 40 ans selon les cas. 19 d'entre eux avaient déjà eu précédemment un ulcère peptique vérifié par endoscopie. 5 avaient déjà été opérés précédemment de leurs ulcères et 5 avaient auparavant été hospitalisés pour ulcère peptique accompagné de saignements.

Au moment de l'inclusion 10 patients avaient un ulcère duodénal déclaré, 7 avaient un ulcère gastrique et 1 avait un ulcère pylorique. 5 patients étaient atteints de duodénite ou de gastrite, mais avec des ulcères guéris. Ces 5 derniers patients avaient subi un traitement intermittent avec un anti-H₂. Tous les malades avaient des symptomes dispeptiques et un test d'uréase à partir de tissu prélevé par biopsie, positif.

### Cicatrisation des ulcères :

4 semaines après le traitement 21 des 23 patients n'avaient plus d'ulcères. Le taux de cicatrisation est de 91,3 % (intervalle de confiance 72,0 à 98,9 %).

### Eradication d'Helicobacter pylori :

20 patients ont obtenu un test de respiration à la ¹⁴C-urée négatif, indiquant l'éradication *d'Helicobacter pylori.* Soit un taux d'éradication de 87,0 % (intervalle de confiance 66,4 à 97,2 %). Une parfaite concordance a été observée entre le test d'uréase et le test de respiration, à l'exception d'un seul malade.

### Effets secondaires :

Parmi les patients ayant pris le traitement jusqu'à la fin, 4 ont été atteints de nausées, 16 de diarrhées et 4 de douleurs abdominales. Néanmoins dans l'ensemble les effets secondaires ont été classés comme "légers" c'est à dire ne perturbant les activités journalières habituelles, à l'exception de seulement 2 malades pour lesquels les effets ont été considérés comme "sévères".

Il a ainsi été possible d'obtenir des taux d'éradication d'*Helicobacter pylori* et de cicatrisation des ulcères similaires aux taux des meilleurs traitements, mais avec un traitement de plus courte durée que pour certains traitements connus et surtout des effets secondaires largement réduits puisque seulement 2 malades sur 24 on rencontré des effets secondaires nuisant à leur activité journalière habituelle. (Dans des études analogues, par administration d'autres antibactériens des diarrhées modérées à sévères avaient été observées chez 41,5 % des malades ce qui est considérablement plus élevé que 1 sur 23, soit 4,4 % dans la présente étude).

La présente invention concerne également les compositions pharmaceutiques comprenant la spiramycine éventuellement en association avec le métronidazole, destinées à la prévention ou au traitement des désordres gastrointestinaux dans lesquels intervient *Helicobacter pylori.*

Comme compositions pour administration orale, peuvent être utilisés des comprimés, des gélules, des pilules, des poudres des lyophilisats ou des granulés. Ces compositions peuvent contenir le(les) principe(s) actif(s) à l'état pur ou éventuellement en association avec un ou plusieurs diluants, lubrifiants ou adjuvants compatibles et pharmaceutiquement acceptables.

A titre d'exemple les compositions peuvent contenir des excipients comme l'amidon, la dextrine, la gélatine, la polyvinylpyrrolidone, l'alumine hydratée, la silice hydratée, le phosphate dicalcique, le stéarate de magnésium, le sorbitol, le mannitol, le lactose, le saccharose, l'acide citrique ....

Il est bien entendu que le médecin adaptera la posologie au sujet à traiter. Plus particulièrement la posologie journalière peut être comprise entre 0,75 et 10 millions U.I. de spiramycine éventuellement en association avec 125 à 1500 mg de métronidazole en 2 à 3 prises.

Les compositions selon l'invention seront utilisées dans tous les cas de prévention ou de traitement des désordres gastrointestinaux dans lesquels intervient *Helicobacter pylori.* Notamment les ulcères gastriques, ulcères duodénaux, ulcères de l'oesophage, gastrites, ulcères pré-pyloriques, dyspepsie non ulcéreuse, cancers gastriques.

## Revendications

1. Utilisation de la spiramycine éventuellement en association avec le métronidazole pour la préparation d'un médicament destiné à la prévention ou au traitement des désordres gastrointestinaux dans lesquels intervient *Helicobacter pylori.*

2. Utilisation de la spiramycine éventuellement en association avec le métronidazole pour la préparation d'un médicament destiné à la prévention ou au traitement des désordres gastrointestinaux dans lesquels intervient *Helicobacter pylori* ledit traitement étant effectué avant, après ou simultanément au traitement par un agent anti-ulcère.

3. Composition pharmaceutique contenant la spiramycine en association avec le métronidazole pour une utilisation selon la revendication 1 ou 2.

4. Composition pharmaceutique selon la revendication 3 contenant un agent anti-ulcère et de la spiramycine éventuellement en association avec du métronidazole.

5. Composition pharmaceutique selon la revendication 4 **caractérisée en ce que** l'agent anti-ulcère est choisi parmi les antiacides, les anti-H₂ et les inhibiteurs de pompe à protons.

## Patentansprüche

1. Verwendung von Spiramycin, gegebenenfalls in Assoziation mit Metronidazol, für die Herstellung eines Arzneimittels zur Vorbeugung oder Behandlung von gastro-intestinalen Störungen, bei denen *Helicobacter pylori* einbezogen ist.

2. Verwendung von Spiramycin, gegebenenfalls in Assoziation mit Metronidazol, für die Herstellung eines Arzneimittels zur Vorbeugung oder Behandlung von gastro-intestinalen Störungen, bei denen *Helicobacter pylori* einbezogen ist, wobei die genannte Behandlung vor, nach oder gleichzeitig mit der Behandlung durch ein Anti-Ulcus-Mittel durchgeführt wird.

3. Pharmazeutische Zusammensetzung, enthaltend Spiramycin in Assoziation mit Metronidazol, für eine Verwendung nach Anspruch 1 oder 2.

4. Pharmazeutische Zusammensetzung nach Anspruch 3, enthaltend ein Anti-Ulcus-Mittel und Spiramycin, gegebenenfalls in Assoziation mit Metronidazol.

5. Pharmazeutische Zusammensetzung nach Anspruch 4, **dadurch gekennzeichnet, daß** das Anti-Ulcus-Mittel unter den Anti-Säure-Mitteln, den Anti-H₂ und den Inhibitoren der Protonenpumpe ausgewählt wird.

## Claims

1. Use of spiramycin, optionally in combination with metronidazole, for the preparation of a medicinal product intended for the prevention or treatment of gastrointestinal disorders involving *Helicobacter pylori.*

2. Use of spiramycin, optionally in combination with metronidazole, for the preparation of a medicinal product intended for the prevention or treatment of gastrointestinal disorders involving *Helicobacter pylori,* the said treatment being carried out before, after or at the same time as the treatment with an anti-ulcer agent.

3. Pharmaceutical composition containing spiramycin in combination with metronidazole, for a use according to Claim 1 or 2.

4. Pharmaceutical composition according to Claim 3, containing an anti-ulcer agent and spiramycin, optionally in combination with metronidazole.

5. Pharmaceutical composition according to Claim 4, **characterized in that** the anti-ulcer agent is chosen from antacids, anti-H₂ agents and proton pump inhibitors.
